# EUROPEAN PATENT SPECIFICATION

(11) **EP 2 608 715 B1**
(45) Date of publication and mention of the grant of the patent: **22.06.2016**
(21) Application number: 11820380.1
(22) Date of filing: 12.08.2011
(51) Int. Cl.: A61B 5/05

(54) **IMPLANTABLE BIOSENSOR DEVICE AND METHODS OF USE THEREOF**
IMPLANTIERBARE BIOSENSORVORRICHTUNG UND VERFAHREN ZU IHRER VERWENDUNG
DISPOSITIF BIOCAPTEUR IMPLANTABLE ET LEURS PROCÉDÉS D'UTILISATION

(30) Priority: 24.08.2010 US 376339 P
(43) Date of publication of application: 03.07.2013
(73) Proprietor: Microchips, Inc., Waltham, Massachusetts 02451 (US)
(72) Inventor: COPPETA, Jonathan, R., Windham NH 03087 (US); FARRA, Robert, Acton MA 01720 (US); HILTS, Kenneth, L., Merrimac MA 01860 (US); SHEPPARD, Norman, F., Jr., New Ipswich NH 03071 (US)
(74) Representative: Patentanwaltskanzlei Matschnig & Forsthuber OG
(86) International application number: PCT/US2011/047607
(87) International publication number: WO 2012/027137

(56) References cited:
- US-A1- 2004 121 486
- US-A1- 2005 051 440
- US-A1- 2005 096 587
- US-A1- 2005 115 832
- US-A1- 2007 213 611
- US-A1- 2008 200 789
- US-A1- 2008 302 659
- US-A1- 2010 042 075

## Description

### Cross-Reference to Related Applications

The present application claims the benefit of U.S. Provisional Application No. 61/376,339, filed on August 24, 2010, which is incorporated herein by reference in its entirety.

### Field of Invention

This invention relates generally to sensor devices, and more particularly to electrochemical sensors and sensor arrays, which may be packaged for medical implant applications.

### Background

U.S. Patent No. 7,604,628, U.S. Patent No. 6,551,838, and U.S. Patent Application Publication No. 2005/0096587 to Santini, et al. describe sensors and sensor components stored in one or an array of discrete, protective reservoirs, which can be selectively and actively opened to expose the sensor or component to a fluid environment outside of the reservoir. In one example, the sensor is a chemical sensor and part of an implantable medical device for detecting glucose or other analytes *in vivo.* In one case, these reservoirs have one or more defined openings that are closed off by one or more reservoir caps, or lids, that can be disintegrated by selective application of an electric current through the caps.

It would be desirable to provide improved sensor devices. For example, it would be advantageous to improve sensing accuracies, increase production and operation efficiencies, and extend the useful life of the sensor(s), while minimizing medical implant device size for ease of implantation in a patient. It would be desirable to package sensors in ways that improve sensing accuracies, increase production and operation efficiencies, extend the useful life of the sensor(s), and/or reduce medical implant device size for ease of implantation in a patient.

### Summary

In one aspect, a sensor device is provided for detecting the presence or concentration of an analyte in a fluid. In one embodiment, the device includes a structural body which comprises a first reservoir that has a first opening in the structural body; a working electrode located within the first reservoir; a catalyst covering at least a portion of the working electrode; an oxygen-generating auxiliary electrode located within the first reservoir; and at least one reservoir cap closing the first opening to isolate the working electrode and the auxiliary electrode within the first reservoir and to prevent an analyte outside of the first reservoir from contacting the catalyst. The device may further include means for selectively rupturing or displacing the at least one reservoir cap to pennit the analyte from outside of the first reservoir to contact the catalyst. The catalyst may comprise an enzyme-containing layer and a membrane. The enzyme containing layer may comprise glucose oxidase or other enzymes useful in medical diagnostics. The structural body may include an array of reservoirs, each of which houses a sensor for detecting the presence or the concentration of the analyte. In a particular embodiment, the sensor device is part of an implantable medical device.

In another aspect, methods are provided for monitoring one or more biochemical species in a patient, such as for diagnosis and/or treatment of the patient. In one embodiment, a method is provided for *in vivo* monitoring of a patient's glucose level. The method may include i) implanting in the patient a device which comprises an array of two or more reservoirs, each reservoir having at least one opening closed off by a reservoir cap and each reservoir containing a working electrode, a membrane and glucose oxidase covering at least a portion of the working electrode, and an oxygen-generating auxiliary electrode; ii) disintegrating the reservoir cap of a first of the two or more reservoirs to permit glucose to enter the first reservoir; iii) generating oxygen using the oxygen-generating auxiliary electrode of the first reservoir; and iv) using the working electrode of the first reservoir to oxidize hydrogen peroxide produced by the reaction of the oxygen with glucose in the presence of the glucose oxidase, and thereby to detect the level of endogenous glucose in the patient. The oxygen generated may be in an amount effective to ensure that glucose is the limiting reactant in the reaction with oxygen. The current for the electrolysis may be provided by alternately charging and discharging a capacitor that is electrically connected to the oxygen-generating electrode. In one embodiment, the method further includes disintegrating the reservoir cap of a second of the two or more reservoirs to permit endogenous glucose to enter the second reservoir; generating oxygen using the oxygen-generating auxiliary electrode of the second reservoir, and using the working electrode of the second reservoir to oxidize hydrogen peroxide produced by the reaction of the oxygen with glucose in the presence of the glucose oxidase, and thereby to detect the level of endogenous glucose in the patient.

### Brief Description of the Drawings

**FIG. 1** is a cross-sectional view of one embodiment of a sensor device in accordance with the present description.
**FIG. 2** is a cross-sectional view of another embodiment of a sensor device in accordance with the present description.
**FIGS. 3-19** are plan views (looking into the reservoirs) of various electrode configurations of sensor devices in accordance with the present description.
**FIGS. 20-21** are cross-sectional views of an embodiment of a sensor device, showing the reservoir in a hermetically sealed state (**FIG. 20**) and then in a opened, operational state (**FIG. 21**).
**FIG. 22** is plan view (looking into the reservoirs) of one embodiment of a sensor device comprising an array of low sensitivity sensors and an array of high sensitivity sensors.

### Detailed Description

Electrochemical sensor devices are provided in configurations/packages to address one or more of the needs described above. For example, sensor electrodes have been designed and arranged to improve sensor accuracy, enhance sensor useful life, and permit reduced implant device dimensions.

In one aspect, a sensor device is provided for detecting the presence or concentration of an analyte in a fluid. The sensor device may comprise a structural body which comprises a first reservoir that has at least one opening, a working electrode located within the first reservoir, a catalyst covering at least a portion of the working electrode, an oxygen-generating auxiliary electrode located within the first reservoir, and at least one reservoir cap closing the at least one opening to isolate the working electrode and the auxiliary electrode within the first reservoir and to prevent an analyte outside of the first reservoir from contacting the catalyst. Certain biological environments may not have adequate oxygen concentration levels for reliable sensor operation. It has been determined that an oxygen-generating auxiliary electrode advantageously may be located in the reservoir with the working electrode to provide an on-demand supply of oxygen and enhance the sensing functionality of the working electrode.

In certain embodiments, the sensor device includes an array of two or more of these sensors, i.e., electrode-containing reservoirs. In this way, the sensor device can serve as a continuous monitor by utilizing each sensor in succession as their operational lifetimes are reached. The most straightforward approach to construction of the monitor is to contain each individual sensor within one reservoir. However, there may be reasons to consider different configurations where (i) the individual electrodes making up a sensor are not contained within the same reservoir, or (ii) a subset of the electrodes making up a sensor are not contained in a reservoir at all. These may be important, for example, when trying to reduce the size of an implanted sensing device.

In some embodiments, the oxygen-generating auxiliary electrode generates oxygen by electrolysis of water or other oxidizable species present in the reservoir. In a preferred embodiment, the oxygen-generating auxiliary electrode is employed in a glucose sensor. The oxygen generated by the electrode diffuses to a catalyst, such as glucose oxidase, and reacts with glucose in the presence of the catalyst to produce hydrogen peroxide, which is oxidized at the working electrode. Although oxygen is present in blood and interstitial fluid along with the glucose, sensing accuracy may be enhanced by providing additional oxygen with the auxiliary electrode, thus ensuring that glucose is the limiting reactant.

A cathode for sinking the electrolysis current from the oxygen-generating electrode may be located in the reservoir with oxygen-generating electrode. The cathode may advantageously reduce the potential pH changing effects resulting from diffusion of the electrolysis reaction products out of the reservoir.

The oxygen-generating electrode (or electrolysis anode) may be operated in a potentiostatic, potentiodynamic, galvanostatic or galvanodynamic modes. The anode may operate continuously or in a pulsed mode. In some embodiments, current for electrolysis is provided by alternately charging and discharging a capacitor that is electrically connected, e.g., shunted, to the oxygen-generating electrode. In such an embodiment, the cycle duration of the charging and discharging sequence may be controlled by the control system of the sensor device such that the length of the cycle is short enough to provide sufficient oxygen concentration at the working electrode throughout the duration of sensor measurement. In some embodiments, the cycle time, measured between charging events of the capacitor may be about 5 seconds to about 5 minutes, or more preferably about 5 seconds to about 3 minutes, or even more preferably about 10 seconds to about 2 minutes, or most preferably about 20 seconds to about 1 minute. Various factors may affect the preferred capacitor cycle time, such as the location of implantation of the sensor (e.g., whether the sensor is exposed to turbulent or quiescent fluid flow), the local oxygen concentration, the sensor oxygen requirements, as well as the electrode and reservoir sizes and configurations.

The potential of the oxygen-generating electrode may be controlled to generate a sufficient quantity of oxygen to provide for a stable sensor output. Without being limited to any one theory, it is expected that the oxygen generation rate will follow the Bulter-Volmer expression which relates the electrode current density to an exponential function of the electrochemical reaction overpotential. The circuitry may maintain the potential relative to a suitable reference electrode such as a silver-silver chloride (Ag/AgCl) electrode so as to better control the oxygen generation rate. In some embodiments, the magnitude of the potential may be about 2.4 volts to about 5.0 volts, or more preferably, about 2.4 to about 4.0 volts, or even more preferably about 2.8 to about 3.1 volts. Various factors may affect the preferred electrolysis potential, such as the location of implantation of the sensor (e.g., whether the sensor is exposed to turbulent or quiescent fluid flow), the local oxygen concentration, the sensor oxygen requirements, the possibility of generating of products that may be sensed at the working electrode, and the electrode and reservoir sizes and configurations.

The control system for the sensor device may utilize software to control the potential magnitude (e.g., the oxygen generation rate), the duration of electrolysis, and/or the electrolysis scheduling. For example, a servo loop may be configured to check the capacitor voltage on a programmable time period, e.g., every 15 seconds. If the capacitor voltage is greater than the set voltage, the control system may shunt the capacitor and check the capacitor voltage every 2 ms until the capacitor voltage is less than the set voltage. If the capacitor voltage is less than set voltage, the control system may charge the capacitor and check the voltage of the capacitor ever 5 ms until the capacitor is above the set voltage. Electrolysis can also be controlled as a function of the analyte concentration available at the sensor. Therefore, the oxygen generation rate can be a function of the oxygen required to ensure appropriate stoichiometry. Higher analyte concentrations will require higher oxygen rates, and vice versa. Other control methods are also envisioned.

### Sensor Device

In certain embodiments, the electrochemical sensor devices include a structural body which comprises at least one reservoir, or more typically an array of two or more discrete reservoirs, each reservoir having at least one opening in the structural body; one or more of the electrodes of one or more chemical sensors located within the reservoir; at least one discrete reservoir cap closing the at least one opening of each reservoir to isolate the electrode(s) (and catalyst, if present) that are located within the reservoir and to prevent external environmental components (e.g., an analyte) outside of the reservoir from contacting the electrode therein; and activation means for rupturing or displacing the reservoir cap to permit the external environmental components (e.g., an analyte) to contact the electrode. In exemplary embodiments, the discrete reservoir caps are in register with predefined openings in the structural body. In various embodiments, the array may include from 4 to 400, from 10 to 200, from 20 to 100, or any number of these sensors/reservoirs in spaced relation to one another in a closely packed array.

The term "biosensor" as used herein is not to be construed as being limited to sensors for medical applications. The sensors device structures described herein may be useful in non-medical applications.

The sensor device may comprise an amperometric biosensor that directly measures current produced by the oxidation or reduction of an electroactive species at a suitably polarized electrode. An amperometric biosensor can include three electrodes: a working electrode, a reference electrode, and a counter electrode. Suitable instrumentation may be used to maintain the working electrode at a controlled potential relative to the reference electrode. In some cases, the amperometric biosensor may be constructed with two electrodes where the functions of the reference electrode and the counter electrode are combined. The biosensors' biological recognition element may be······though not in all embodiments-an enzyme for which the analyte of interest is a biochemical substrate. Amperometric sensors can exploit the fact that many co-substrates or products of the reaction catalyzed by the enzyme are electroactive. These sensors serve to measure the concentration of a co-substrate or product in the enzyme layer. In the presence of the analyte, the concentration of the co-substrate will decrease and that of the product will increase. The resulting change in sensor current can be related to the analyte concentration through a suitable calibration. Representative examples of suitable enzymes may include glucose oxidase, glucose dehydrogenase, NADH oxidase, uricase, urease, creatininase, sarcosine oxidase, creatinase, creatine kinase, creatine amidohydrolase, cholesterol esterase, cholesterol oxidase, glycerol kinase, hexokinase, glycerol-3-phosphate oxidase, lactate oxidase, lactate dehydrogenase, alkaline phosphatase, alanine transaminase, aspartate transaminase, amylase, lipase, esterase, gamma-glutamyl transpeptidase, L-glutamate oxidase, pyruvate oxidase, diaphorase, bilirubin oxidase, and mixtures thereof. An amperometric biosensor could be constructed without an enzyme layer, for example if the biosensor is configured to measure oxygen.

It is advantageous to contain the working electrode in a sealed reservoir for selective exposure (such as at the precise time the electrode is needed for a particular sensor to function) in order to protect the working electrode against (i) fouling of the outer layer of the sensor by proteins and cells which influence transport of analyte to the enzyme layer, (ii) degradation of the enzyme by the hydrogen peroxide produced by oxidase enzymes, (iii) degradation of polymer layers, for example, the hydrolysis of ester linkages of polyurethane membranes, and (iv) degradation processes mediated by cells of the immune system (e.g., macrophages, foreign body giant cells). In addition, hermetically sealed reservoirs enable the environment (e.g., inert gas atmosphere, humidity) inside the sealed reservoir to be controlled, which may lead to a longer lifetime of the sensor.

In one embodiment, the sensor device is a glucose biosensor based on the enzyme glucose oxidase. The enzyme-catalyzed conversion of analyte (e.g., glucose) yields a reaction product (e.g., hydrogen peroxide) that is redox active. (Alternatively, the catalytic activity of the enzyme may result in the consumption of a redox-active co-substrate, such as oxygen in the glucose sensor.) The oxidation or reduction of the redox active compound at a suitably polarized electrode produces a current that can be related back to the analyte concentration.

The sensor device may comprise a structural body that has a first reservoir provided therein. The structural body may have on its exterior a first opening fluidly connected to the reservoir. A working electrode and an auxiliary electrode may be contained in the first reservoir. A catalyst may cover at least a portion of the working electrode. The auxiliary electrode may be configured to generate oxygen so that the oxygen generated by the auxiliary electrode diffuses toward the working electrode. The sensor device may further comprise at least one reservoir cap closing the first opening to isolate the working electrode and the auxiliary electrode within the first reservoir and to prevent an analyte outside of the first reservoir from contacting the catalyst.

The particular sensors packaged as described herein may take a variety of different forms. In some embodiments, the sensors are tailored for glucose sensing. In a certain embodiment, the present packaged sensor device may include electrodes and glucose sensor chemistries, e.g., catalysts, as described in U.S. Patent No. 6,881,551 to Heller et al. or as described in U.S. Patent No. 4,890,620 to Gough et al.

In a preferred embodiment, the sensor device utilizes three sensing electrodes and an auxiliary oxygen-generating electrode. The three sensing electrodes include working, counter and reference electrodes. The working electrode is where the desired analyte is oxidized or reduced, yielding the sensor current. The reference electrode is used to establish the potential in the solution; the external circuitry (potentiostat) maintains a specified potential between the reference electrode and the working electrode. The reference electrode desirably is in close proximity to the working electrode to reduce any resistive (IR) potential drops, which may change the working electrode potential. The counter electrode sinks or sources the working electrode current. The counter electrode may be equal in area to or larger in area than the working electrode in order to reduce the current density and overpotential at the counter electrode.

In certain embodiments of the present devices and methods, the working electrode and oxygen-generating electrode are located within a reservoir that is sealed and can be selectively unsealed or opened. In a preferred embodiment, the reference electrodes are also protected by locating them within one or more reservoirs. This may be a preferred configuration for an implantable sensor device. The reference electrode may be in close proximity to the working electrode, e.g., in the same reservoir as the working electrode, and may be protected from environmental degradation by the reservoir cap.

The working electrode in the reservoir includes, e.g., is covered completely or at least partially by, an appropriate catalyst. The reference electrode may or may not be covered by the catalyst. In one embodiment, it may be preferable or simpler to deposit the catalyst over both electrodes, and in this way the reference electrode may be considered to be measuring the environment seen by the working electrode. However this may not be desirable for certain embodiments where the composition of the reference electrode is such that it reacts or interferes with catalyst. For example, silver ions from a silver/silver chloride reference electrode may inhibit glucose oxidase activity. In such embodiments, the catalyst preferably is applied to cover only the working electrode. It can facilitate depositing a catalyst over an electrode to first surround the electrode with a barrier as conventionally known, for example, as shown in U.S. Patent No. 5,376,255 to Gumbrecht, et al.

The nature and placement of the counter electrode outside of the reservoir may be varied. For example, it may be located on a lower substrate portion, coplanar with the working and reference electrodes, or it could be on a surface of an upper substrate portion. (The term "upper substrate portion" as used herein may be referred to in the art as a "microchip" or "microchip portion," as this substrate may include electronic circuitry for operation/actuation of reservoir cap disintegration.) In one embodiment, the portions of the reservoir caps remaining after activation, e.g., following electrothermal ablation, and the electrical traces connecting to the reservoir caps may be utilized as the counter electrode. In another embodiment, the counter electrode is located on a surface of the upper substrate portion of the reservoir device, but is electrically isolated from the reservoir caps or traces connected to the reservoir caps. In yet another embodiment, a counter electrode "external" to the sensor and reservoir substrates, such as a wire lead or the electronics case may be used. It may be advantageous to locate the counter electrode outside of the reservoir, to minimize the interaction between redox reactions occurring at the counter electrode and reactions taking place at the working electrode. A reason to separate the electrodes is that oxygen may be consumed at the counter electrode which may otherwise limit the amount of oxygen available in the enzyme layer at the working electrode for glucose oxidation.

In another embodiment, the reference electrode is provided in a separate reservoir than the working electrode and oxygen-generating electrode. This may be less desirable from the standpoint of having the reference electrode close to the working electrode, but may be desirable where the lifetime of the reference electrode is considerably greater than the working electrode, such that a single reference electrode could be used with a succession of working electrodes. In one embodiment, a single reference electrode (and a single counter electrode) may be used with a two working electrodes operating simultaneously, in a configuration under control of a bipotentiostat. In another embodiment, a single reference electrode (and a single counter electrode) may be used with more than two working electrodes operating simultaneously. Similarly, one counter electrode may be used with more than one working electrode.

Examples of various embodiments of the sensor devices are illustrated in **FIGS**. **1-****21.** These are not drawn to scale. The shapes and dimensions of the electrodes, the reservoirs, the reservoir openings, the catalyst and membranes, the substrates, and the bonding layers, if any, may be varied as needed to accommodate device specifications and manufacturing design constraints. It is to be understood from the figures that show only a single reservoir, that, in certain embodiments, a sensor device would include a structural body comprising an array of multiple such representative reservoirs/sensors.

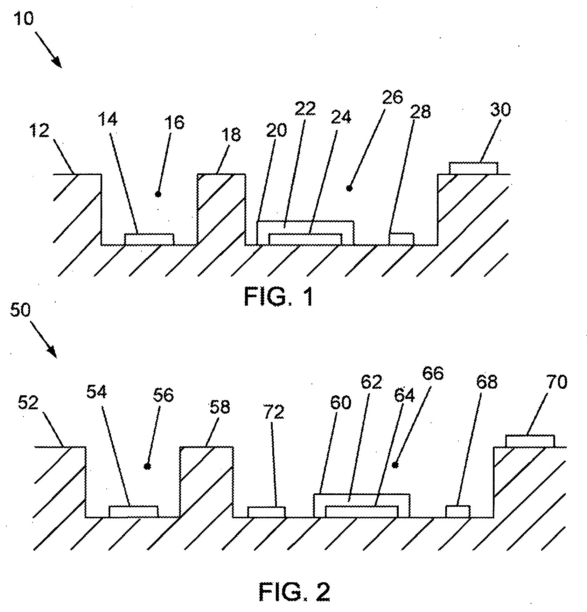

**FIG. 1** shows one embodiment of a sensor device **10**. The device **10** generally includes a structural body or substrate **12**. Reservoir **16** and **26** are formed in, or otherwise defined by, the substrate **12,** and are separated by a wall **18**. Although only two reservoirs are shown, an array of reservoirs may be provided. Each reservoir may be, for example, identical and discrete, although other configurations are possible. For example, the reservoir **16** that is adjacent to the reservoir **26** may be of a different size.

The sensor device **10** also includes a working electrode **24,** an oxygen-generating auxiliary electrode **28**, a reference electrode **14** and a counter electrode **30.** As shown, the working electrode **24** and auxiliary electrode **28** are disposed within the reservoir **26,** the reference electrode **14** is disposed within reservoir **16**, and the counter electrode **30** is provided on the substrate **12** outside of the reservoirs **26** and **16**.

The device **10** further includes catalyst **22** located in the reservoir **26.** The catalyst **22** may include, for example, an enzyme and a membrane **20.** The membrane **20** may comprise one or more polymer layers, such as those useful as semi-permeable membranes to permit passage of an analyte of interest therethrough while excluding certain other molecules. The catalyst **22** may be deposited directly onto the working electrode **24.** Although not shown, the catalyst **22** may also be deposited on the reference electrode **14**, so that the reference electrode **14** is exposed to (i.e. "sees") the same environment as the working electrode **24.** In the illustrated embodiment, the catalyst **22** is not deposited on the reference electrode **14**

Although not shown in the present illustration, one or more reservoir caps cover the openings of the reservoir **26** and the reservoir **16**. For example, a single reservoir cap could cover both reservoir **16** and reservoir **26** or each of the reservoirs **16** and **26** may be covered by a discrete reservoir cap. In another example, reservoir **16** and/or reservoir **26** each has two or more predefined openings, which may be defined by reservoir cap support structures. These multiple openings per reservoir may each be closed off by its own reservoir cap. In either of these examples, the one or more reservoir caps may be electrically conductive, and traces or leads may be provided for directing electric current through the reservoir cap.

The sensor device **10** also includes power and control systems (not shown) that power and control disintegration of the one or more reservoir cap and operatively couple to the electrodes. The power and control systems may be provided in a hardwired or wireless manner, for example, as described in U.S. Patent No. 7,226,442 and U.S. Patent Application publication No. 2005/0096587.

The illustrated embodiment of the device **10** includes a single set of electrodes **14, 24,28**, and **30** associated with a pair of reservoirs **16** and **26**, forming a sensor. In other embodiments, the device **10** may include an array of reservoirs **16** and **26.** For example, the device **10** may include a number of discrete reservoirs that may be opened sequentially, such as one or two at a time, as a preceding exposed sensor becomes fouled and a fresh sensor is needed.

**FIG. 2** shows an alternative embodiment of a sensor device **50**, in which a cathode **72** is located in a single reservoir **66** with the working electrode **64** and the oxygen-generating auxiliary electrode **68**. The reference electrode **54** is provided in a separate reservoir **56** that is separated from the reservoir **66** by a wall **58**. The cathode **72** serves to sink the electrolysis current generated by the oxygen-generated by the auxiliary electrode **68.** By placing the cathode **72** in the reservoir **66** with the auxiliary electrode **68**, the cathode **72** may advantageously reduce the effect of the electrolysis reaction products outside of the reservoir, e.g., by preventing or limiting pH change. The sensor device **50** may also include a counter electrode **70** outside of the reservoirs **56** and **66**.

Although not shown in the present illustration, one or more reservoir caps may cover the openings of the reservoir **66** and the reservoir **56.** For example, a single reservoir cap could cover both reservoir **56** and reservoir **66** or each of the reservoirs **56** and **66** may be covered by a discrete reservoir cap. The one or more reservoir caps may be electrically conductive, and traces or leads may be provided for directing electric current through the reservoir cap.

The device **50** further includes catalyst **62** located in the reservoir **66**. The catalyst **62** may include, for example, an enzyme and a membrane **60.** The membrane **60** may comprise one or more polymer layers, such as those useful as semi-permeable membranes to permit passage of an analyte of interest therethrough while excluding certain other molecules. The catalyst **62** may be deposited directly onto the working electrode **64.** Although not shown, the catalyst **62** may also be deposited on the reference electrode **54,** so that the reference electrode **54** is exposed to (i.e. "sees") the same environment as the working electrode **24.** In the illustrated embodiment, the catalyst **62** is not be deposited on the reference electrode **54.**

The sensor device **50** also includes power and control systems (not shown) that power and control disintegration of the one or more reservoir cap and operatively couple to the electrodes as described with reference to **FIG. 1**

The illustrated embodiment of the device **50** includes a single set of electrodes **54, 64,68, 70**, and **72** associated with a pair of reservoirs **56** and **66,** forming a sensor. In other embodiments, the device **50** may include an array of reservoirs **56** and **66.** For example, the device **50** may include a number of discrete reservoirs that may be opened sequentially, such as one or two at a time, as a preceding exposed sensor becomes fouled and a fresh sensor is needed.

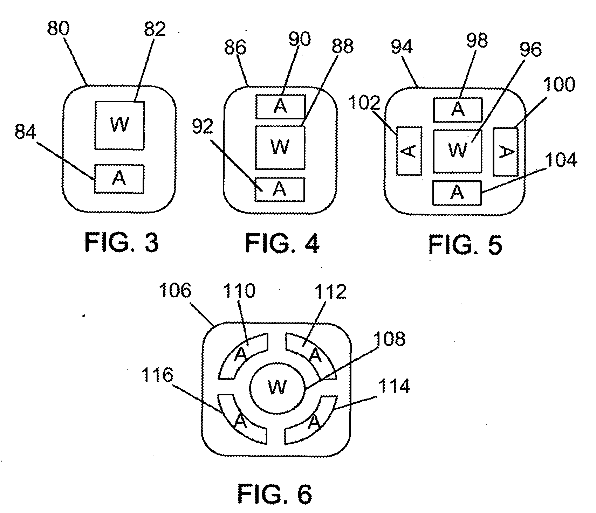

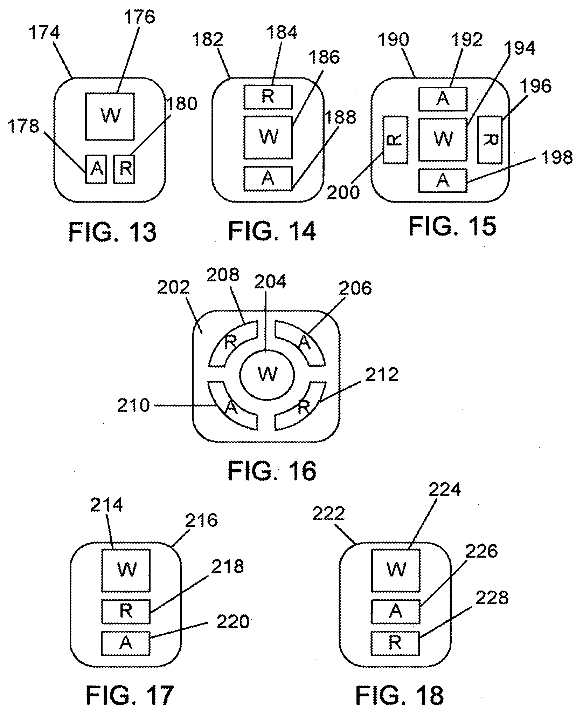

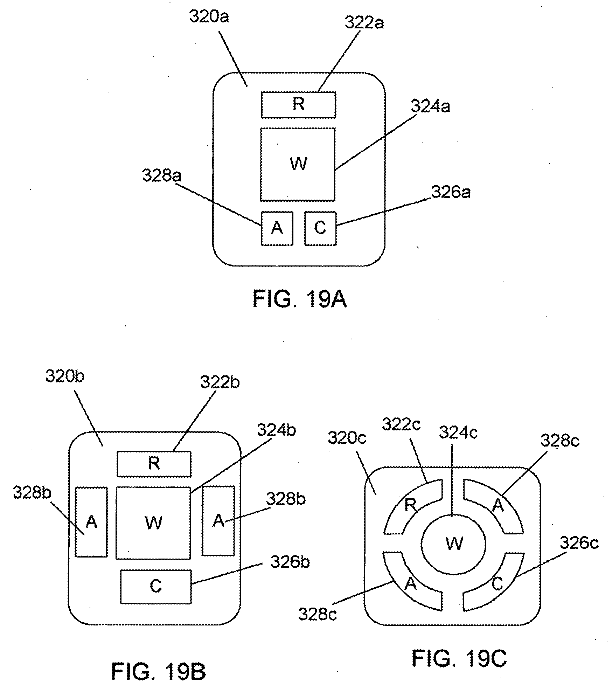

**FIGs. 3-19** illustrate variations of electrode configurations that may be used in a sensor device, such as the sensor devices of **FIGs**. **1**. and **2**. Each of **FIGs. 3-19** illustrate the electrode configuration within a single reservoir. It should be noted that the sensor may comprise an array of such reservoirs and electrodes. The array may comprise a plurality of identical reservoirs with identical electrode configurations.

**FIG. 3** shows an embodiment of a sensor having a working electrode **82** and an oxygen-generating auxiliary electrode **84** in a common reservoir **80.** **FIG. 4** shows an embodiment of a sensor having a working electrode **88** and two oxygen-generating auxiliary electrodes **90** and **92** in a common reservoir **86.** **FIG. 5** shows an embodiment of a sensor having a working electrode **96** and four oxygen-generating auxiliary electrodes **98**,**100**, **102,** and **104** in a common reservoir **94**. **FIG**. **6** shows an embodiment of a sensor having a circular working electrode **108** and four auxiliary electrodes **110**,**112**,**114** and **116** angularly arranged around the working electrode **108** in a common reservoir **106**. The four auxiliary electrodes **110**, **112**, **114**, and **116** all may be oxygen-generating anodes or one or more of the electrodes may be cathodes.

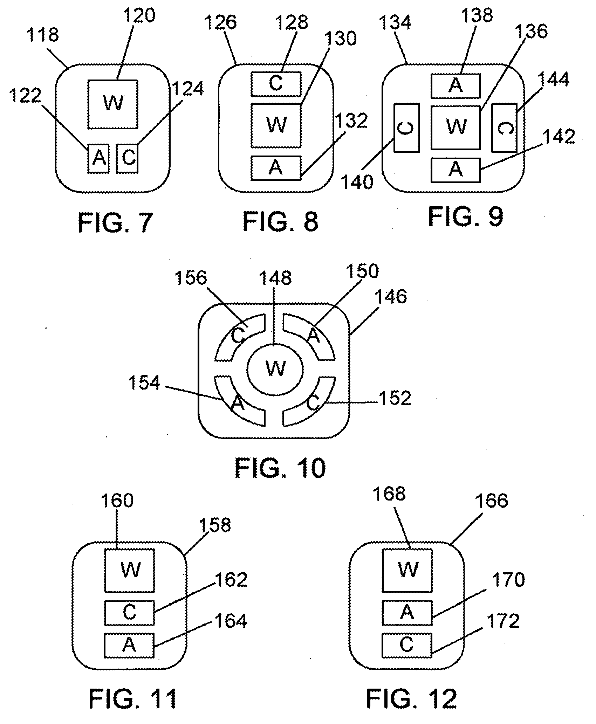

**FIG.** 7 shows an embodiment of a sensor having a working electrode 1**20,** an oxygen-generating auxiliary electrode **122** and a cathode **124** for the auxiliary electrode **122** in a common reservoir **118**. In this embodiment, the auxiliary electrode **122** and the cathode **124** are positioned side-by-side next to one side of the working electrode **120.**

**FIG**. **8** shows an embodiment of a sensor having a working electrode **130,** an oxygen-generating auxiliary electrode **132** and a **cathode 128** for the auxiliary electrode **132** in a common reservoir **126**. In this embodiment, the auxiliary electrode **132** and the **cathode 128** are positioned on opposite sides of the working electrode **130.**

**FIG. 9** shows an embodiment of a sensor having a working electrode **136**, two oxygen-generating auxiliary electrodes **138** and **142** and two cathodes **140** and **144** for the auxiliary electrodes **138** and **142** in a common reservoir **134**. In this embodiment, the auxiliary electrodes **138** and **142 are** positioned on opposite sides of the working electrode **136**. The cathodes **140** and **144** are also positioned on opposite sides of the working electrode **136**.

**FIG**. **10** shows an embodiment of a sensor having a circular working electrode **148**, two oxygen-generating auxiliary electrodes **150** and **154** and two cathodes **152** and **156** for the auxiliary electrodes **150** and **154** in a common reservoir **146.** In this embodiment, the auxiliary electrodes **150** and **154** and cathodes **140** and **144** are alternately positioned angularly around the working electrode **148.**

**FIG. 11** shows an embodiment of a sensor having a working electrode **160**, an oxygen-generating auxiliary electrode **164** and a cathode **162** for the auxiliary electrode **164** in a common reservoir **158.** In this embodiment, the working electrode **160,** the auxiliary electrode **164** and the cathode **162** are arranged linearly with the cathode **162** being located between the working electrode **160** and the auxiliary electrode **164**.

**FIG**. **12** shows an embodiment of a sensor having a working electrode **168,** an oxygen-generating auxiliary electrode **170** and a cathode **172** for the auxiliary electrode **170** in a common reservoir **166**. In this embodiment, the working electrode **168,** the auxiliary electrode **170** and the cathode **172** are arranged linearly with the auxiliary electrode 170 being located between the working electrode **168** and the cathode **172**.

**FIG**. 13 shows an embodiment of a sensor having a working electrode **176**, an oxygen-generating auxiliary electrode **178** and a reference electrode **180** in a common reservoir **174.** In this embodiment, the auxiliary electrode **178** and the reference electrode **180** are positioned side-by-side next to one side of the working electrode **176.**

**FIG**. **14** shows an embodiment of a sensor having a working electrode **186,** an oxygen-generating auxiliary electrode **188** and a reference electrode **184** in a common reservoir **182.** In this embodiment, the auxiliary electrode **188** and the reference electrode **184** are positioned on opposite sides of the working electrode **186**.

**FIG**. **15** shows an embodiment of a sensor having a working electrode **194,** two oxygen-generating auxiliary electrodes **192** and **198** and two reference electrodes **196** and **200** in a common reservoir **190.** In this embodiment, the auxiliary electrodes **192** and **198** are positioned on opposite sides of the working electrode **194.** The reference electrodes **196** and **200** are also positioned on opposite sides of the working electrode **194.**

**FIG**. **16** shows an embodiment of a sensor having a circular working electrode **204,** two oxygen-generating auxiliary electrodes **206** and **210** and two reference electrodes **208** and **212** in a common reservoir **202.** In this embodiment, the auxiliary electrodes **206** and **210** and the reference electrodes **208** and **212** are alternately positioned angularly around the working electrode **204.**

**FIG**. **17** shows an embodiment of a sensor having a working electrode **214,** an oxygen-generating auxiliary electrode **220** and a reference electrode **218** in a common reservoir **216.** In this embodiment, the working electrode **214**, the auxiliary electrode **220,** and the reference electrode **218** are arranged linearly with the reference electrode **218** being located between the working electrode **214** and the auxiliary electrode **220.**

**FIG**. **18** shows an embodiment of a sensor having a working electrode **224,** an oxygen-generating auxiliary electrode **226** and a reference electrode **228** in a common reservoir **222.** In this embodiment, the working electrode **224**, the auxiliary electrode **226** and the reference electrode **228** are arranged linearly with the auxiliary electrode **226** being located between the working electrode **224** and the reference electrode **228.**

**FIGs**. **19A-C** show embodiments of a sensor having a working electrode **324a/324b/324c,** an oxygen-generating auxiliary electrode **328a/328b/328c,** a cathode **326a/326b/326c** for the auxiliary electrode **328a/328b/328c,** and a reference electrode **322a/322b/322c** in a common reservoir **320a/320b/320c.**

**FIGs**. **20-21** illustrate an embodiment of a sensor device in which electrode components of the sensor device are provided in separate, discrete reservoirs or wells. In the illustrated embodiment, the discrete reservoirs are fluidly connected such that the fluid entering the device through the reservoir openings, e.g., after rupturing or displacing the reservoir cap, may be exposed to the reference electrode and working electrode simultaneously.

**FIGs**. **20** and **21** show an embodiment of a sensor device **330** comprising two substrate portions **332** and **334.** The substrate portion **332** includes joint portions **354** which engage a joint portion **356** of the substrate portion **334** to form a hermetically sealed reservoir **342**. The two substrate portions **332** and **334** may be attached together, for example, by a compression cold weld. Alternatively or additionally, an adhesive may be used to bond substrate portions **332** and **334** together

The substrate portion **332** comprises a plurality of reservoir caps **340,** which hermetically seal the reservoir **342** and its contents from the environment around the device **330.** One or more leads **348** are electrically connected to each of the reservoir caps **340.** In the illustrated embodiment, two leads **348** are electrically connected to each reservoir cap **340** to allow for selective rupturing of the reservoir caps **340** by electrothermal ablation.

The reservoir **342** comprises two, discrete reservoirs or wells **338** and **346.** The reservoir **338** contains a reference electrode **336** and the reservoir **346** contains a working electrode **344** and an oxygen-generating auxiliary electrode **350**. At least a portion of the working electrode **344** is covered by in catalyst **360.** The catalyst **360** may comprise an enzyme, such a glucose oxidase, and a selectively permeable membrane. A counter electrode **352** is provided on an exterior surface of the device **330.**

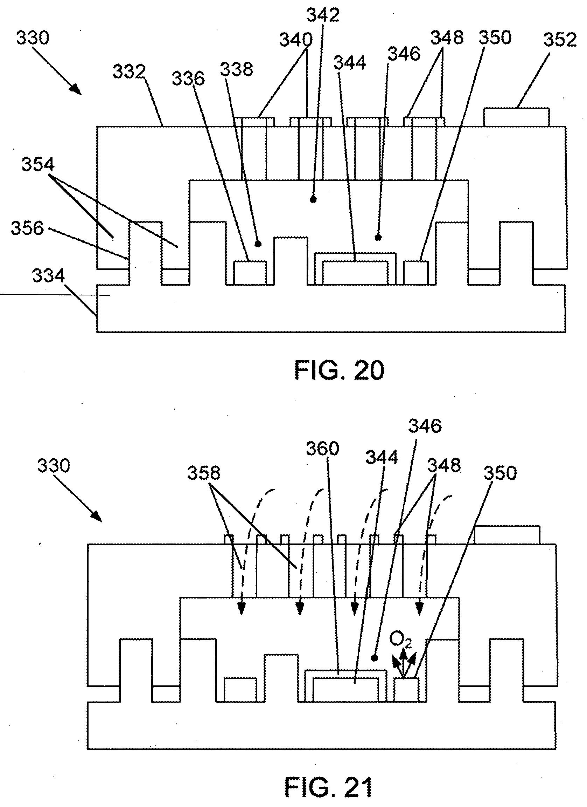

The device **330** also includes power and control systems (not shown) that power and control disintegration of the one or more reservoir cap and operatively couple to the electrodes. The control system may open the reservoir caps **340** at a selected time, as illustrated in **FIG. 21**, by transmitting a current suitable for electrothermally ablating the reservoir caps **340.** This exposes openings **358** in the substrate **332** and allows fluid to fill the reservoirs **342, 338,** and **346.** The control system thereafter my supply a voltage suitable for electrolysis to the oxygen-generating auxiliary electrode **350** to generate oxygen. The oxygen generated by the auxiliary electrode diffuses to the catalyst **360.**

An example of the upper substrate portion **332** and reservoir cap **340** structure is described in U.S. Patent No. 7,604,628, which is incorporated herein by reference. In this way, an individual reservoir may have at least two reservoir openings with a support structure therebetween and closed by two or more reservoir caps covering the openings to control exposure of the electrode(s) within that reservoir. In one embodiment, the substrate comprises at least one reservoir cap support extending over the reservoir contents, wherein the two or more reservoir caps are in part supported by the at least one reservoir cap support. In one embodiment, a sensor device may comprise an array of two or more of such reservoirs, each having multiple reservoir openings. The reservoir cap supports can comprise substrate material, structural material, or coating material, or combinations thereof. The reservoir cap support(s) may be integral with upper substrate portion. Alternatively, the reservoir cap support may be made from a coating or deposited material distinct from the substrate portion. Reservoir cap supports comprising substrate material may be formed in the same step as the reservoirs. MEMS methods, microfabrication, micromolding, and micromachining techniques described herein or known in the art may be used to fabricate the substrate/reservoirs, as well as reservoir cap supports, from a variety of substrate materials.

Although a single reservoir is shown in several of the embodiments described above and illustrated in the appended drawings, it is understood that the sensor device according to the invention includes an array of multiple reservoirs, such as, two, four, ten, twenty, or one hundred reservoirs, each reservoir being associated with a discrete or shared combination of electrodes to form a sensor. Likewise, other combinations of substrate structures, reservoir shapes/sidewall angles, reservoir cap disintegration means, and the like, besides the particular combinations illustrated and described herein, are contemplated.

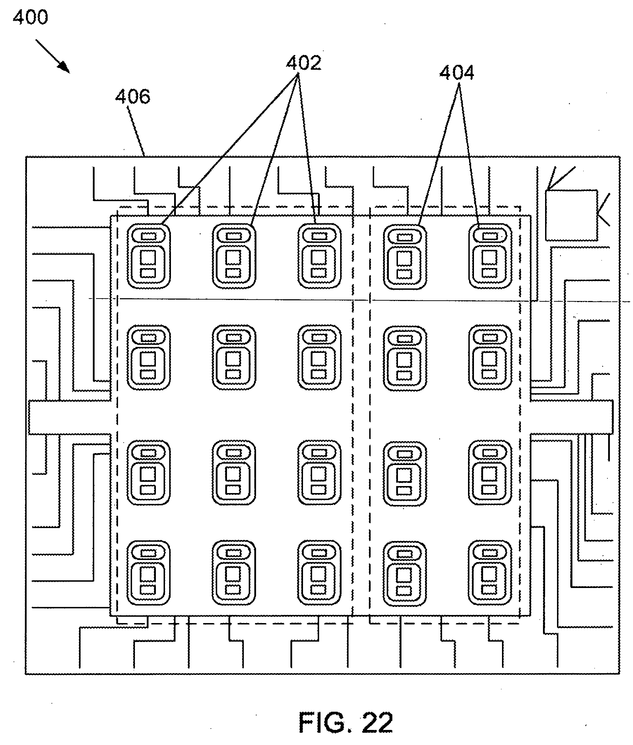

**FIG. 22** illustrates an exemplary embodiment of a sensor device **400** according to the invention. The device **400** includes a substrate **406.** The substrate **406** can be, for example, silicon or another micromachined substrate or combination of micromachined substrates such as silicon and glass, e.g., as described in U.S. Patent Application Publication 2005/0149000 or U.S. Patent 6,527,762. In another embodiment, the substrate comprises multiple silicon wafers bonded together. In yet another embodiment, the substrate comprises a low-temperature co-fired ceramic (LTCC) or other ceramic such as alumina.

The device **400** includes an array of sensors **402** and **404.** In the illustrated embodiment, the device includes an array of twelve low sensitivity sensors **402** (e.g., about 1 nA/100 mg/dL) and an array of eight high sensitivity sensors **404** (e.g., about 15 nA/100 mg/dL). The sensor sensitivity may be controlled by employing a glucose limiting membrane and varying the formulation of the membrane between the low sensitivity sensors **402** and the high sensitivity sensors **404.**

During use, e.g., after implantation, the device's power and control system may selectively actuate the opening the reservoirs of one or more of the low sensitivity sensors **402** and/or one or more of the high sensitivity sensors **404.** For example, the control system may first actuate the opening of a single low sensitivity sensor **402** and the opening of a single high sensitivity sensor **404** by rupturing the reservoir caps covering the sensors. At a later time, such as after the exposed sensors have begun to foul, the control system may actuate the opening of a second low sensitivity sensor **402** and a second high sensitivity sensor **404.**

### Substrate and Reservoirs

In one embodiment, the containment device comprises a body portion, i.e., a substrate, that includes one or more reservoirs for containing reservoir contents sealed in a fluid tight or hermetic manner. As used herein, the term "hermetic" refers to a seal/containment effective to keep out helium, water vapor, and other gases. As used herein, the term "fluid tight" refers to a seal/containment which is not gas hermetic, but which are effective to keep out dissolved materials (e.g., glucose) in a liquid phase. The substrate can be the structural body (e.g., part of a device) in which the reservoirs are formed, e.g., it contains the etched, machined, or molded reservoirs.

In preferred embodiments, the reservoirs are discrete, deformable or non-deformable, and disposed in an array across one or more surfaces (or areas thereof) of the device body. As used herein, the term "reservoir" means a well, a cavity, or a hole suitable for storing or containing a precise quantity of a material, such as sensor or subcomponent. By contrast, the random, interconnected pores of a porous material would not be considered "reservoirs" as that term is used herein. In a one embodiment, the device includes a plurality of the reservoirs located in discrete positions across at least one surface of the body portion. In another embodiment, there is a single reservoir per each reservoir substrate portion; optionally two or more of these portions can be used together in a single device.

Reservoirs can be fabricated in a structural body portion using any suitable fabrication technique known in the art. Representative fabrication techniques include MEMS fabrication processes, microfabrication processes, or other micromachining processes, various drilling techniques (e.g., laser, mechanical, and ultrasonic drilling), and build-up or lamination techniques, such as LTCC. The surface of the reservoir optionally can be treated or coated to alter one or more properties of the surface. Examples of such properties include hydrophilicity/ hydrophobicity, wetting properties (surface energies, contact angles, etc.), surface roughness, electrical charge, release characteristics, and the like. MEMS methods, micromolding, micromachining, and microfabrication techniques known in the art can be used to fabricate the substrate/reservoirs from a variety of materials. Numerous other methods known in the art can also be used to form the reservoirs. See, for example, U.S. Patent No. 6,123,861 and U.S. Patent No. 6,808,522. Various polymer forming techniques known in the art also may be used, e.g., injection molding, thermocompression molding, extrusion, and the like.

In various embodiments, the body portion of the containment device comprises silicon, a metal, a ceramic, a polymer, or a combination thereof. Examples of suitable substrate materials include metals (e.g., titanium, stainless steel), ceramics (e.g., alumina, silicon nitride), semiconductors (e.g., silicon), glasses (e.g., Pyrex™, BPSG), and degradable and non-degradable polymers. Where only fluid tightness is required, the substrate may be formed of a polymeric material, rather than a metal or ceramic which would typically be required for gas hermeticity.

In one embodiment, each reservoir is formed of (i.e., defined in) hermetic materials (e.g., metals, silicon, glasses, ceramics) and is hermetically sealed by a reservoir cap. Desirably, the substrate material is biocompatible and suitable for long-term implantation into a patient. In a preferred embodiment, the substrate is formed of one or more hermetic materials. The substrate, or portions thereof, may be coated, encapsulated, or otherwise contained in a hermetic biocompatible material (e.g., inert ceramics, titanium, and the like) before use. Non-hermetic materials may be completely coated with a layer of a hermetic material. For example, a polymeric substrate could have a thin metal coating. If the substrate material is not biocompatible, then it can be coated with, encapsulated, or otherwise contained in a biocompatible material, such as poly(ethylene glycol), polytetrafluoroethylene-like materials, diamond-like carbon, silicon carbide, inert ceramics, alumina, titanium, and the like, before use. In one embodiment, the substrate is hermetic, that is impermeable (at least during the time of use of the reservoir device) to the contents of the reservoir and to surrounding gases or fluids (e.g., water, blood, electrolytes or other solutions).

The substrate can be formed into a range of shapes or shaped surfaces. It can, for example, have a planar or curved surface, which for example could be shaped to conform to an attachment surface. In various embodiments, the substrate or the containment device is in the form of a planar chip, a circular or ovoid disk, an elongated tube, a sphere, or a wire. The substrate can be flexible or rigid. In one case, the reservoir-based sensors are disposed at the distal end of a flexible lead or catheter for deployment in a body lumen or other tissue site in a patent. In various embodiments, the reservoirs are discrete, non-deformable, and disposed in an array across one or more surfaces (or areas thereof) of an implantable medical device.

The substrate may consist of only one material, or may be a composite or multilaminate material, that is, composed of several layers of the same or different substrate materials that are bonded together. Substrate portions can be, for example, silicon or another micromachined substrate or combination of micromachined substrates such as silicon and glass, e.g., as described in U.S. Patent Application Publication 2005/0149000 or U.S. Patent 6,527,762. In another embodiment, the substrate comprises multiple silicon wafers bonded together. In yet another embodiment, the substrate comprises an LTCC or other ceramic such as alumina. In one embodiment, the body portion is the support for a microchip device. In one example, this substrate is formed of silicon.

In one embodiment, either or both substrates to be bonded may be formed of one or more glasses, which may be particularly useful in embodiments where it is desirable to view or interrogate an object or material that is contained between the sealed substrates, e.g., in a cavity or reservoir. That is, where the substrate can serve as an fluid-tight window. Representative examples of glasses include aluminosilicate glass, borosilicate glass, crystal glasses, etc.

Total substrate thickness and reservoir volume can be increased by bonding or attaching wafers or layers of substrate materials together. The device thickness may affect the volume of each reservoir and/or may affect the maximum number of reservoirs that can be incorporated onto a substrate. The size and number of substrates and reservoirs can be selected to accommodate the size and arrangement of sensor components needed for a particular application, manufacturing limitations, and/or total device size limitations to be suitable for implantation into a patient, preferably using minimally invasive procedures.

In a preferred embodiment for an implantable sensor application using a planar sensor, the substrate preferably is relatively thin, as noted above.

The substrate can have one, two, three or more reservoirs. In various embodiments, tens, hundreds, or thousands of reservoirs are arrayed across the substrate. The number of reservoirs in the device may be determined by the operation life of the individual sensors. For example, a one-year implantable glucose-monitoring device having individual sensors that remain functional for 30 days after exposure to the body may contain at least 12 reservoirs (assuming one sensor per reservoir). In another sensor embodiment, the distance between the sensor surface and the reservoir opening means is minimized, preferably approaching a few microns. In this case, the volume of the reservoir is primarily determined by the surface area of the sensor. For example, the electrodes of a typical enzymatic glucose sensor may occupy a space that is 400 µm by 800 µm.

In one embodiment, the reservoirs are microreservoirs. The "microreservoir" is a reservoir suitable for containing a microquantity of material, such as sensor component materials and sparge gas, if present. In one embodiment, the microreservoir has a volume equal to or less than 500 µL (e.g., less than 250 µL, less than 100 µL, less than 50 µL, less than 25 µL, less than 10 µL, etc.) and greater than about 1 nL (e.g., greater than 5 nL, greater than 10 nL, greater than about 25 nL, greater than about 50 nL, greater than about 1 µL, etc.). The term "microquantity" refers to volumes from 1 nL up to 500 µL. In one embodiment, the microquantity is between 1 nL and 1 µL. In another embodiment, the microquantity is between 10 nL and 500 nL. In still another embodiment, the microquantity is between about 1 µL and 500 µL. The shape and dimensions of the microreservoir can be selected to maximize or minimize contact area between the sensor and the surrounding surface of the microreservoir.

In one embodiment, the reservoir is formed in a 200-micron thick substrate and has dimensions of 1.5 mm by 0.83 mm, for a volume of about 250 nL, not counting the volume that would be taken up by the support structures, which may be about 20 to about 50 microns thick.

In another embodiment, the reservoirs are macroreservoirs. The "macroreservoir" is a reservoir suitable for containing a quantity of material larger than a microquantity. In one embodiment, the macroreservoir has a volume greater than 500 µL (e.g., greater than 600 µL, greater than 750 µL, greater than 900 µL, greater than 1 mL, etc.) and less than 5 mL (e.g., less than 4 mL, less than 3 mL, less than 2 mL, less than 1 mL, etc.).

Unless explicitly indicated to be limited to either micro- or macro-scale volumes/quantities, the term "reservoir" is intended to encompass both.

In one embodiment, the device includes polymeric chips or devices composed of non-silicon based materials that might not be referred to as "microchips."

### Reservoir Cap Supports

Reservoir cap supports can comprise substrate material, structural material, or coating material, or combinations thereof. Reservoir cap supports comprising substrate material may be formed in the same step as the reservoirs. The MEMS methods, microfabrication, micromolding, and micromachining techniques mentioned above could be used to fabricate the substrate/reservoirs, as well as reservoir cap supports, from a variety of substrate materials. Reservoir cap supports comprising structural material may also be formed by deposition techniques onto the substrate and then MEMS methods, microfabrication, micromolding, and micromachining techniques. Reservoir cap supports formed from coating material may be formed using known coating processes and tape masking, shadow masking, selective laser removal techniques, or other selective methods.

A reservoir may have several reservoir cap supports in various configurations over its reservoir contents. For example, one reservoir cap support may span from one side of the reservoir to the opposite side; another reservoir cap support may cross the first reservoir cap support and span the two other sides of the reservoir. In such an example, four reservoir caps could be supported over the reservoir.

In one embodiment for a sensor application (e.g., a glucose sensor), the reservoir (of a device, which can include only one or which may include two or more reservoirs) has three or more reservoir openings and corresponding reservoir caps. The dimensions and geometry of the support structure can be varied depending upon the particular requirements of a specific application.

### Reservoir Caps

As used herein, the term "reservoir cap" refers to a membrane, thin film, or other structure suitable for separating the contents of a reservoir from the environment outside of the reservoir, but which is intended to be removed or disintegrated at a selected time to open the reservoir and expose its contents. In one embodiment, a discrete reservoir cap completely covers a single opening in a reservoir. In this embodiment, the reservoir may have one, or more than one, opening. If more than one opening, each opening is covered by its own reservoir cap, which may be selectively disintegrated simultaneously with or separately from disintegration of the other reservoir caps associated with the reservoir. In another embodiment, a reservoir cap covers two or more openings at once; these opening may be to the same reservoir or to two difference reservoirs. In preferred actively controlled devices, the reservoir cap includes any material that can be disintegrated or permeabilized in response to an applied stimulus (e.g., electric field or current, magnetic field, change in pH, or by thermal, chemical, electrochemical, or mechanical means). Examples of suitable reservoir cap materials include gold, titanium, platinum, tin, silver, copper, zinc, alloys, and eutectic materials such as gold-silicon and gold-tin eutectics. Any combination of passive or active barrier layers can be present in a single device.

In one embodiment, the reservoir caps are electrically conductive and non-porous. In a preferred embodiment, the reservoir caps are in the form of a thin metal film. In another embodiment, the reservoir caps are made of multiple metal layers, such as a multi-layer/laminate structure of platinum/titamum/platinum. For example, the top and bottom layers could be selected for adhesion layers on (typically only over a portion of) the reservoir caps to ensure that the caps adhere to/bonds with both the substrate area around the reservoir openings, reservoir cap supports, and a dielectric overlayer. In one case, the structure is titanium/platinum/titanium/platinum/titanium, where the top and bottom layers serve as adhesion layers, and the platinum layers provide extra stability/biostability and protection to the main, central titanium layer. The thickness of these layers could be, for example, about 300 nm for the central titanium layer, about 40 nm for each of the platinum layers, and between about 10 and 15 nm for the adhesion titanium layers.

### Control Means for Disintegrating or Permeabilizing the Reservoir Cap

The containment device includes control means that facilitates and controls reservoir opening, e.g., for disintegrating or permeabilizing the reservoir caps at a select time following sealing of the reservoirs as described herein. The control means comprises the structural component(s) and electronics (e.g., circuitry and power source) for powering and for controlling the time at which exposure of the sensor is initiated.

The control means can take a variety of forms. In one embodiment, the reservoir cap comprises a metal film that is disintegrated by electrothermal ablation as described in U.S. Patent No. 7,510,551, and the control means includes the hardware, electrical components, and software needed to control and deliver electric energy from a power source (e.g., battery, storage capacitor) to the selected reservoir caps for actuation, e.g., reservoir opening. For instance, the device can include a source of electric power for applying an electric current through an electrical input lead, an electrical output lead, and a reservoir cap connected therebetween in an amount effective to disintegrate the reservoir cap. Power can be supplied to the control means of the multi-cap reservoir system locally by a battery, capacitor, (bio)fuel cell, or remotely by wireless transmission, as described for example in U.S. Patent No. 7,226,442. A capacitor can be charged locally by an on-board battery or remotely, for example by an RF signal or ultrasound.

In one embodiment, the control means includes an input source, a microprocessor, a timer, a demultiplexer (or multiplexer). The timer and (de)multiplexer circuitry can be designed and incorporated directly onto the surface of the substrate during fabrication. In another embodiment, some of the components of the control means are provided as a separate component, which can be tethered or untethered to the reservoir portion of the device. For instance, the controller and/or power source may be physically remote from, but operably connected to and/or in communication with, the multi-cap reservoir device. In one embodiment, the operation of the multi-cap reservoir system will be controlled by an on-board (e.g., within an implantable device) microprocessor. In another embodiment, a simple state machine is used, as it typically is simpler, smaller, and/or uses less power than a microprocessor.

In certain embodiments, the structural body (which sometimes may be referred to as the "substrates"), the reservoirs, the reservoir caps, and the activation means for rupturing or displacing the reservoir cap, and how these various components may be packaged together to form hermetically sealed reservoir devices, are described, for example, in U.S. Patent No. 6,527,762 (which describes thermal means for reservoir cap rupture); U.S. Patent No. 6,551,838; U.S. Patent No. 6,976,982 (which describes flexible substrate/body structures); U.S. Patent Application Publication No. 2006/0115323 (which describes hermetic sealed reservoir structures and compression cold weld sealing methods); U.S. Patent No. 7,510,551 (which describes electrothermal ablation means for reservoir cap disintegration); U.S. Patent No. 7,604,628 (which describes reservoir/structural body designs with multiple discrete reservoir caps closing off a single reservoir opening); and U.S. Patent Application Publication No. 2005/0096587. These patents and patent applications are incorporated herein by reference.

In a certain embodiment, the reservoir cap is formed of a conductive material, such as a metal film, through which an electrical current can be passed to electrothermally ablate it, as described in U.S. Patent No. 7,510,551 to Uhland, et al. In this embodiment, the reservoir cap itself serves both as a structural barrier for isolating the contents of the reservoir from substances outside of the reservoir and as the heating element. Representative examples of suitable reservoir cap materials include gold, copper, aluminum, silver, platinum, titanium, palladium, various alloys (e.g., Au/Si, Au/Ge, Pt--Ir, Ni--Ti. Pt--Si, SS 304, SS 316), and silicon doped with an impurity to increase electrical conductivity, as known in the art. The reservoir cap may be in the form of a multi-layer structure, such as a multi-layer/laminate structure of platinum/titanium/platinum.

The reservoir cap may be operably (i.e., electrically) connected to an electrical input lead and to an electrical output lead, to facilitate How of an electrical current through the reservoir cap. When an effective amount of an electrical current is applied through the leads and reservoir cap, the temperature of the reservoir cap is locally increased due to resistive heating, and the heat generated within the reservoir cap increases the temperature sufficiently to cause the reservoir cap to be electrothermally ablated (ruptured or disintegrated). The heating may be rapid and substantially instantaneous upon application of an electric current through the reservoir cap, such that no substantial heating of substances (e.g., sensor chemistry, patient tissues) adjacent to the reservoir cap occurs.

In one embodiment, the reservoir cap and the conductive leads are formed of the same material, and the temperature of the reservoir cap increases locally under applied current because the reservoir cap is suspended in a medium that is less thermally conductive than the substrate. Alternatively, the reservoir cap and conductive leads are formed of the same material, and the reservoir cap has a smaller cross-sectional area in the direction of electric current flow, where the increase in current density through the reservoir cap causes an increase in localized heating. The reservoir cap alternatively can be formed of a material that is different from the material forming the leads, wherein the material forming the reservoir cap has a different electrical resistivity, thermal diffusivity, thermal conductivity, and/or a lower melting temperature than the material forming the leads. Various combinations of these embodiments can be employed. For example, the reservoir cap and the input and output leads may be designed to provide (i) an increase in electrical current density in the reservoir cap relative to the current density in the input and output leads, upon the application of electrical current, (ii) that the material forming the reservoir cap has a different electrical resistivity, thermal diffusivity, thermal conductivity, and/or a lower melting temperature than the material forming the input and output leads, or (iii) both (i) and (ii).

In another embodiment, the reservoir cap is configured as an anode and the device further includes a cathode, along with electrical circuitry, a power source, and controls for applying an electric potential between the cathode and anode in an electrically conductive fluid environment (e.g., *in vivo*) to cause the reservoir cap to disintegrate as described in U.S. Patent No. 5,797,898 to Santini Jr. et al. In still another embodiment, the reservoir cap is configured to rupture by heating using a separate resistive heating element, which may be located either inside the reservoir or outside the reservoir, generally adjacent to the reservoir cap, as described for example in U.S. Patent No. 6,527,762 to Santini Jr. et al.

### Using the Sensor Devices

The sensor devices and systems described herein can be used in a wide variety of applications. Preferred applications include biosensing, such as sensing for glucose. In a preferred embodiment, the multi-cap reservoir system is part of an implantable medical device. The implantable medical device can take a wide variety of forms and be used in a variety of therapeutic and/or diagnostic applications.

The reservoirs may be opened as needed (depending, for example, upon fouling of the sensor) or as dictated by a predetermined schedule. In a particular embodiment, the reservoirs comprise a glucose sensor, which may, for instance, comprise glucose oxidase immobilized on an electrode in the reservoir and coated with one or more permeable/semi-permeable membranes. Because the enzyme could lose its activity when exposed to the environment (e.g., the body) before its intended time of use, the sealed reservoir serves to protect the enzyme until it is needed.

It is understood that the sensor devices described herein may be used as or adapted for inclusion in (e.g., included as part of) a medical device, such as an implantable medical device. In a non-limiting example, the implantable medical device may include an array of several sensors for long term sensing applications, such as glucose sensing, which would be useful for example in the management of a patient's diabetes. In another embodiment, the sensor device may be integrated into the end portion of a medical catheter or other flexible lead intended for insert to the body of a patient for therapeutic or diagnostic purposes.

It is contemplated that a sensor device, such as an implantable medical device or other medical device, may include various combinations of the sensor types and configurations described herein. For example, a single device, such as an implantable device, may include multiple different sensors. In one particular example, such a device may include an amperometric sensor (e.g., configured as a glucose sensor).

It is also understood that the sensor devices described herein may be used as or adapted for inclusion in non-medical devices and systems. For example, the sealed devices described herein have numerous *in vitro* and commercial diagnostic applications, such as analytical chemistry and medical diagnostics. Also, the sensors may be used as environmental sensors, which may have a number of particular applications. In one case, the devices may be used to sense heavy metals or other pollutants in bodies of water, such as lakes and streams. In another case, the devices may be used to detect biological weapon agents. Such devices could be adapted to be fixed or mobile, for use in public venues, as a wearable device on first responders, in public transit systems, airports, on military vehicles, etc.

All documents cited in the Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

Modifications and variations of the methods and devices described herein will be obvious to those skilled in the art from the foregoing detailed description. Such modifications and variations are intended to come within the scope of the appended claims.

## Claims

1. A sensor device (400) for detecting the presence or concentration of an analyte in a fluid comprising:
a structural body (406) which comprises an array of reservoirs, each of the reservoirs having a first opening in the structural body;
a sensor located within each of the reservoirs, the sensor comprising a working electrode, a catalyst covering at least a portion of the working electrode and an oxygen-generating auxiliary electrode;
at least one reservoir cap closing the first opening to isolate the working electrode and the auxiliary electrode within each of the reservoirs and to prevent an analyte outside of the first reservoir from contacting the catalyst; and
a means for rupturing or displacing the at least one reservoir cap of each of the reservoirs to permit the analyte from outside to contact the catalyst,
wherein the array of reservoirs comprises a first array of low sensitivity sensors (402) and a second array of high sensitivity sensors (404).

2. The sensor device of claim 1, wherein the means for rupturing or displacing comprises:
a pair of conductive leads electrically connected to the at least one reservoir cap, the at least one reservoir cap comprising an electrically conductive material; and
a power source for applying an electrical current through the at least one reservoir cap via the pair of conductive leads,
wherein the pair of conductive leads and power source are adapted to rupture the at least one reservoir cap by electrothermal ablation.

3. The sensor device of claim 1, wherein the means for rupturing or displacing effects a phase change in the reservoir cap.

4. The sensor device of any one of claims 1 to 3, further comprising a reference electrode.

5. The sensor device of claim 4, wherein the reference electrode is located within each of the reservoirs.

6. The sensor device of claim 5, wherein each of the reservoirs comprises a wall separating the reference electrode from the working electrode.

7. The sensor device of any one of claims 1 to 6, further comprising a counter electrode.

8. The sensor device of claim 7, wherein the counter electrode is located outside of the reservoirs.

9. The sensor device of any one of claims 1 to 8, wherein the catalyst comprises an enzyme-containing layer and a membrane.

10. The sensor device of claim 9, wherein the enzyme is selected from the group consisting of glucose oxidase, glucose dehydrogenase, NADH oxidase, uricase, urease, creatininase, sarcosine oxidase, creatinase, creatine kinase, creatine amidohydrolase, cholesterol esterase, cholesterol oxidase, glycerol kinase, hexokinase, glycerol-3-phosphate oxidase, lactate oxidase, lactate dehydrogenase, alkaline phosphatase, alanine transaminase, aspartate transaminase, amylase, lipase, esterase, gamma-glutamyl transpeptidase, L-glutamate oxidase, pyruvate oxidase, diaphorase, bilirubin oxidase, and mixtures thereof.

## Patentansprüche

1. Sensorvorrichtung (400) zum Detektieren der Anwesenheit oder der Konzentration eines Analyten in einem Fluid, umfassend:
einen Baukörper (406), der eine Anordnung von Reservoirs aufweist, wobei jedes Reservoir eine erste Öffnung im Baukörper aufweist;
einen Sensor, der innerhalb eines jeden Reservoirs angeordnet ist, wobei der Sensor eine Arbeitselektrode, einen Katalysator, der zumindest einen Abschnitt der Arbeitselektrode bedeckt, und eine Sauerstoff generierende Hilfselektrode umfasst;
zumindest eine Reservoirabdeckung, die die erste Öffnung schließt, um die Arbeitselektrode und die Hilfselektrode innerhalb eines jeden Reservoirs zu isolieren und um zu verhindern, dass ein Analyt, der sich außerhalb des ersten Reservoirs befindet, mit dem Katalysator in Kontakt kommt; und
Mittel zum Aufbrechen oder Verdrängen der zumindest einen Reservoirabdeckung eines jeden Reservoirs, um dem Analyten von außen den Kontakt mit dem Katalysator zu gestatten,
wobei die Anordnung an Reservoirs eine erste Anordnung an Sensoren niedriger Empfindlichkeit (402) und eine zweite Anordnung an Sensoren hoher Empfindlichkeit (404) umfasst.

2. Sensorvorrichtung nach Anspruch 1, wobei die Mittel zum Aufbrechen oder Verdrängen aufweisen:
ein Paar Leitungen, die mit der zumindest einen Reservoirabdeckung elektrisch verbunden sind, wobei die zumindest eine Reservoirabdeckung ein elektrisch leitfähiges Material umfasst; und
eine Stromquelle zum Anlegen eines elektrischen Stroms durch die zumindest eine Reservoirabdeckung über das Paar Leitungen,
wobei das Paar Leitungen und die Stromquelle dazu eingerichtet sind, die zumindest eine Reservoirabdeckung mittels elektrothermischer Ablation aufzubrechen.

3. Sensorvorrichtung nach Anspruch 1, wobei die Mittel zum Aufbrechen oder Verdrängen einen Phasenwechsel in der Reservoirabdeckung bewirken.

4. Sensorvorrichtung nach einem der Ansprüche 1 bis 3, welche überdies eine Referenzelektrode umfasst.

5. Sensorvorrichtung nach Anspruch 4, wobei die Referenzelektrode innerhalb eines jeden Reservoirs angeordnet ist.

6. Sensorvorrichtung nach Anspruch 5, wobei jedes Reservoir eine Trennwand aufweist, welche die Referenzelektrode von der Arbeitselektrode trennt.

7. Sensorvorrichtung nach einem der Ansprüche 1 bis 6, welche überdies eine Gegenelektrode umfasst.

8. Sensorvorrichtung nach Anspruch 7, wobei die Gegenelektrode außerhalb der Reservoirs angeordnet ist.

9. Sensorvorrichtung nach einem der Ansprüche 1 bis 8, wobei der Katalysator eine Enzymhältige Schicht und einen Membran umfasst.

10. Sensorvorrichtung nach Anspruch 9, wobei das Enzym aus der Gruppe bestehend aus Glukoseoxidase, Glukosedehydrogenase, NADH-Oxidase, Uricase, Urease, Kreatininase, Sarkosinoxidase, Kreatinase, Kreatinkinase, Kreatinamidohydrolase, Cholesterinesterase, Cholesterinoxidase, Glycerinkinase, Hexokinase, Glycerin-3-Phosphat-Oxidase, Laktatoxidase, Laktatdehydrogenase, Alkalische Phosphatase, Alanintransaminase, Aspartattransaminase, Amylase, Lipase, Esterase, Gamma-Glutamyltranspeptidase, L-Glutamatoxidase, Pyruvatoxidase, Diaphorase, Bilirubinoxidase und deren Mischungen ausgewählt ist.

## Revendications

1. - Dispositif capteur (400) pour détecter la présence ou la concentration d'un analyte dans un fluide, comprenant :
un corps structural (406) qui comprend un réseau de réservoirs, chacun des réservoirs ayant une première ouverture dans le corps structural ;
un capteur situé à l'intérieur de chacun des réservoirs, le capteur comprenant une électrode de travail, un catalyseur recouvrant au moins une partie de l'électrode de travail et une électrode auxiliaire générant de l'oxygène ;
au moins un capuchon de réservoir fermant la première ouverture pour isoler l'électrode de travail et l'électrode auxiliaire à l'intérieur de chacun des réservoirs et empêcher un analyte à l'extérieur du premier réservoir d'entrer en contact avec le catalyseur ; et
un moyen de rupture ou de déplacement du au moins un capuchon de réservoir de chacun des réservoirs pour permettre à l'analyte provenant de l'extérieur d'entrer en contact avec le catalyseur,
le réseau de réservoirs comprenant un premier réseau de capteurs à faible sensibilité (402) et un second réseau de capteurs à sensibilité élevée (404).

2. - Dispositif capteur selon la revendication 1, dans lequel le moyen de rupture ou de déplacement comprend :
une paire de fils conducteurs électriquement connectés à l'au moins un capuchon de réservoir, le au moins un capuchon de réservoir comprenant une matière conductrice de l'électricité ; et
une source d'énergie destinée à appliquer un courant électrique à travers le au moins un capuchon de réservoir par l'intermédiaire de la paire de fils conducteurs,
la paire de fils conducteurs et la source d'énergie étant adaptés pour rompre le au moins un capuchon de réservoir par ablation électrothermique.

3. - Dispositif capteur selon la revendication 1, dans lequel le moyen de rupture ou de déplacement effectue un changement de phase dans le capuchon de réservoir.

4. - Dispositif capteur selon l'une quelconque des revendications 1 à 3, comprenant en outre une électrode de référence.

5. - Dispositif capteur selon la revendication 4, dans lequel l'électrode de référence est située à l'intérieur de chacun des réservoirs.

6. - Dispositif capteur selon la revendication 5, dans lequel chacun des réservoirs comprend une paroi séparant l'électrode de référence de l'électrode de travail.

7. - Dispositif capteur selon l'une quelconque des revendications 1 à 6, comprenant en outre une contre-électrode.

8. - Dispositif capteur selon la revendication 7, dans lequel la contre-électrode est située à l'extérieur des réservoirs.

9. - Dispositif capteur selon l'une quelconque des revendications 1 à 8, dans lequel le catalyseur comprend une couche à teneur en enzyme et une membrane.

10. - Dispositif capteur selon la revendication 9, dans lequel l'enzyme est choisie dans le groupe consistant en la glucose oxydase, la glucose déshydrogénase, la NADH oxydase, l'uricase, l'uréase, la créatininase, la sarcosine oxydase, la créatinase, la créatine kinase, la créatine amidohydrolase, la cholestérol estérase, la cholestérol oxydase, la glycérol kinase, l'hexokinase, la glycérol-3-phosphate oxydase, la lactate oxydase, la lactate déshydrogénase, la phosphatase alcaline, l'alanine transaminase, l'aspartate transaminase, l'amylase, la lipase, l'estérase, la gamma-glutamyl transpeptidase, la L-glutamate oxydase, la pyruvate oxydase, la diaphorase, la bilirubine oxydase et leurs mélanges.
